# EUROPEAN PATENT SPECIFICATION

(11) **EP 1 180 368 B1**
(45) Date of publication and mention of the grant of the patent: **18.04.2007**
(21) Application number: 00935503.3
(22) Date of filing: 31.05.2000
(51) Int. Cl.: A61K 38/22, A61K 9/14, A61K 9/19

(54) **FREEZE DRIED HGF PREPARATIONS**
GEFRIERGETROCKNETE HGF-PRÄPARATIONEN
PREPARATIONS LYOPHILISEES DE HGF

(30) Priority: 31.05.1999 JP 15176999
(43) Date of publication of application: 20.02.2002
(73) Proprietor: Mitsubishi Chemical Corporation, Minato-ku Tokyo 108-0014 (JP)
(72) Inventor: CHIBA, M., Mitsubishi Chem. Corp., Kashima Plant, Kashima-gun, Ibaraki 314-0255 (JP)
(74) Representative: polypatent
(86) International application number: PCT/JP2000/003506
(87) International publication number: WO 2000/072873

(56) References cited:
- EP-A- 0 838 221
- EP-A1- 0 456 188
- WO-A-98/28007
- JP-A- 6 040 938

## Description

### Technical Field

The present invention relates to a lyophilized preparation comprising a hepatocyte growth factor.

### Background Art

Hepatocyte growth factor (abbreviated occasionally as "HGF" hereafter in the specification) is a protein having a proliferating activity of hepatocytes and its existence in various animal species is known. HGFs having different amino acid sequences have been reported. Human hepatocyte growth factor (abbreviated occasionally as "hHGF" hereafter in the specification) was found from plasma of a fulminant hepatitis patient by Daikuhara et al. (Japanese Patent Unexamined Publication (Kokai) No. 63-22526). The amino acid sequence of the hHGF protein and the gene (cDNA) sequence encoding said protein were found by Kitamura et al. (Japanese Patent Unexamined Publication No. 3-72883). A method for producing the hHGF protein and a transformant using said cDNA have been reported (Japanese Patent Unexamined Publication No. 3-285693). Under the circumstances, mass production of the hHGF protein becomes possible and its application as a medicament is expected.

hHGF is a kind of glycoprotein, which is a heterodimer consisting of a subunit having a molecular weight of about 80-90 kDa in a non-reduced state or about 52-56 kDa in a reduced state and β subunit having a molecular weight of about 30-36 kDa. Besides the activity as hepatic cell growth factor, hHGF has various biological activities such as a scatter factor (SF) activity, renal tubular epithelial cell growth factor activity, damaged tissue repair factor activity and vascular endothelial cell growth factor activity, and the protein is expected to be developed as medicaments for therapeutic treatment of liver diseases, kidney diseases, cranial nerve disorders, hair growth promoters, wound healing agents, antitumor therapeutic agents and the like.

Pharmaceutical preparations of HGF are described in WO90/10651 and Japanese Patent Unexamined Publication Nos. 6-247872 and 9-25241. The aforementioned WO90/10651 discloses an aqueous preparation of deletion-type HGF (TCF) in which five amino acid residues are deleted from HGF, and the publication teaches that albumin, human serum, gelatin, sorbitol, mannitol, xylitol and the like stabilize TCF in an aqueous solution. Japanese Patent Unexamined Publication No. 6-247872 discloses an injection containing TCF at a high concentration of 5-10 mg/mL in which a basic amino acid or the like coexists with TCF. This publication refers to the solubility of TCF in an aqueous solution and discloses an aqueous solution containing TCF at a high concentration. The basic amino acid (lysine, arginine) is used as a "solubilizing aid" in the injection.

However, the aqueous HGF preparation rapidly decreases the solubility of HGF at a neutral pH and has a problem of progress of aggregation, cloudiness and gelation when stored at a low temperature or room temperature for several days. Further, the preparation has low physicochemical stability, for example, formation of degradation products and aggregates, and also has poor stability as a pharmaceutical preparation, for example, reduce of biological activity. Therefore, the preparation is not suitable for a long-term storage from a viewpoint of biological activity. Furthermore, the aqueous HGF preparation may cause aggregation, cloudiness, and gelation due to foaming or the like after shaking and stirring, which leads to decreases of quality of a pharmaceutical preparation and drug efficacy during long-term storage, distribution and transportation. Therefore, a lyophilized preparation is preferred as an HGF preparation.

Japanese Patent Unexamined Publication No. 9-25241 discloses a lyophilized preparation of HGF (TCF). However, unlike the present invention, the patent publication teaches that a lyophilized preparation comprising HGF (TCF) at a high concentration that is stable over a long period can be provided by using a citrate as a buffering agent and glycine, alanine, sorbitol, mannitol or the like as a stabilizing agent. However, due to the citric acid used as a buffering agent in the lyophilized preparation, a pH of a redissolved preparation will be in an acidic condition. Further, the resulting solution has a high osmotic pressure, which causes problems of pain at administration by injection, or inflammatory reaction and hemolysis at an administration site and the like.

HGF is a substance having extremely potent physiological activities, and when used as a medicament, the substance needs to be provided in the clinical filed as a pharmaceutical preparation having a very low concentration. Studies by the inventors of the present invention revealed that, as for the lyophilized HGF (TCF) preparation comprising glycine or alanine described in Japanese Patent Unexamined Publication No. 9-25241, only a little formation of aggregates was observed during storage when the lyophilized preparation was produced from an aqueous solution containing HGF at a high concentration, whilst aggregate formation was observed during storage when a preparation was produced in the presence of glycine or alanine by lyophilizing an aqueous solution containing HGF at a low concentration, which is desirable for clinical application (generally, HGF is contained at a concentration lower than 5 mg/mL, for example, about 2 mg/mL). Accordingly, glycine or alanine described in Japanese Patent Unexamined Publication No. 9-25241 is useful as a stabilizing agent when HGF is lyophilized at a high concentration, however, the amino acid is not sufficient as a stabilizing agent when HGF is lyophilized at a low concentration. It has therefore been desired to develop a method for producing a lyophilized preparation that hardly forms aggregates and has excellent stability in long-term storage by using an aqueous solution containing HGF at a low concentration.

### Disclosure of the Invention

An object of the present invention is to provide a lyophilized HGF preparation that can produce an aqueous solution containing HGF at a low concentration. More specifically, the object of the present invention is to provide a lyophilized HGF preparation that has excellent storage stability and is free from aggregation, cloudiness, gelation or the like upon redissolution. Another object of the present invention is to provide a lyophilized preparation that has a favorable cake forming property during lyophilization and excellent re-solubility. Yet another object of the present invention is to provide the preparation having a pH and an osmotic pressure ratio desirable as an injection.

The inventors of the present invention conducted various studies to achieve the foregoing objects. As a result, they found that, when a solution containing HGF at a concentration lower than 5 mg/mL was lyophilized in the presence of a stabilizing agent, sodium chloride and a buffering agent, a lyophilized preparation having a favorable cake forming property, solubility and long-term storage stability was successfully produced, and that aggregates were not formed in the production of the lyophilized preparation as well as during storage of said preparation, and the lyophilized preparation had extremely high stability. Further, they also found that, an aqueous solution prepared from the lyophilized preparation was free from aggregation, cloudiness, gelation or the like, and the aqueous solution containing the low concentration of HGF successfully exert sufficient clinical effectiveness. The present invention was achieved on the basis of the above findings.

The present invention thus provides:
a lyophilized preparation comprising a hepatocyte growth factor, a stabilising agent selected from the group consisting of arginine, lysine, histidine, glutamic acid, aspartic acid and a pharmacologically acceptable salt thereof for preventing formation of an aggregate of the hepatocyte growth factor, sodium chloride, and a buffering agent, **characterized in that** the lyophilized preparation is prepared from an aqueous solution containing the hepatocyte growth factor at a concentration lower than 5 mg/mL and/or the lyophilized preparation is used for preparing an aqueous solution containing the hepatocyte growth factor at a concentration lower than 5 mg/mL by redissolution.

These lyophilized HGF preparations do not give HGF aggregates during lyophilization and long-term storage after the lyophilization and have excellent stability. Further, they have characteristic features that aggregation, cloudiness, gelation or the like does not occur in an aqueous solution prepared from the lyophilized preparation, and, even after storage of the aqueous solution, aggregates are hardly formed.

Usually, it is preferred that an aqueous solution used to prepare a lyophilized preparation in a vial and an aqueous solution prepared by dissolving the resulting lyophilized preparation in the vial contain the same concentration of an active ingredient. Therefore, the preparation of the present invention can preferably be produced by lyophilizing an aqueous solution containing HGF at a concentration lower than 5 mg/mL in a vial or an ampoule. Further, as for the preparation of the present invention, a pH of the aqueous solution before lyophilization and/or the aqueous solution obtained after redissolution is preferably in the range of 5 to 6.5, which is desirable as an injection. Also as for the preparation of the present invention, the aqueous solution before lyophilization and/or the aqueous solution obtained after redissolution preferably have an osmotic pressure desirable as an injection, for example, almost isotonic in living bodies or osmotic pressure ratio acceptable as an injection (1 to 2).

According to preferred embodiments of the present invention, there are provided the aforementioned lyophilized HGF preparation, wherein the stabilizing agent is selected from the group consisting of arginine, lysine, histidine, glutamic acid, aspartic acid, and pharmacologically acceptable salts thereof; the aforementioned lyophilized HGF preparation, wherein the stabilizing agent is selected from the group consisting of arginine, lysine, histidine, glutamic acid, aspartic acid and pharmacologically acceptable salts thereof; the aforementioned lyophilized HGF preparation, wherein the stabilizing agent is selected from the group consisting of arginine, lysine, histidine and pharmacologically acceptable salts thereof; and the aforementioned lyophilized HGF preparation, wherein the stabilizing agent is selected from the group consisting of arginine, lysine and pharmacologically acceptable salts thereof. These stabilizing agents are preferably added to the preparation in an amount sufficient to prevent formation of a aggregate of HGF during lyophilization and/or storage after the lyophilization.

Further, according to other preferred embodiments of the present invention, there are provided the aforementioned lyophilized HGF preparation, wherein the buffering agent is a phosphoric acid salt; the aforementioned lyophilized HGF preparation, which further contains a surface active agent; the aforementioned lyophilized HGF preparation, wherein the surface active agent is a nonionic surface active agent; and the aforementioned lyophilized preparation, wherein the nonionic surface active agent is a polyoxyethylene ether surface active agent.

From another aspect of the present invention, there is provided a stabilizing agent for HGF used to lyophilize an aqueous solution containing HGF at a concentration lower than 5 mg/mL, which is selected from the group consisting of arginine, lysine, histidine, glutamic acid, aspartic acid, and pharmacologically acceptable salts thereof. Preferred stabilizing agents are selected from the group consisting of arginine, lysine, histidine, glutamic acid, aspartic acid, and pharmacologically acceptable salts thereof. Particularly preferred stabilizing agents are selected from the group consisting of arginine, lysine, and pharmacologically acceptable salts thereof. These stabilizing agents can prevent formation of a aggregate of HGF during lyophilization of an aqueous solution containing HGF at a concentration of lower than 5 mg/mL and during storage after the lyophilization.

The present invention further provides a lyophilized HGF preparation, which can be obtained by lyophilizing an aqueous solution containing the aforementioned stabilizing agent and HGF at a concentration lower than 5 mg/mL and is used to prepare an aqueous solution containing HGF at a concentration lower than 5 mg/mL by redissolution. A preferred preparation of the aforementioned lyophilized preparation can be obtained by lyophilizing an aqueous solution containing the stabilizing agent (preferably, a stabilizing agent selected from a group consisting of arginine, lysine and pharmacologically acceptable salts thereof), HGF at a concentration lower than 5 mg/mL, sodium chloride and a buffering agent in a vial.

### Best Mode for Carrying out the Invention

The type of HGF contained in the lyophilized preparation of the present invention is not particularly limited. For example, natural HGF may be isolated from humors or tissues derived from mammals such as human and rat, which are known to contain HGF, or cells that spontaneously produce HGF. A recombinant HGF obtained by introducing cDNA of said growth factor into cells by gene recombination technique may also be used. Examples of hosts for producing a recombinant HGF include *Escherichia coli, Bacillus subtilis*, yeast, filamentous fungi, plant cells, insect cells, animal cells and the like. Specific examples of the recombinant HGF include those obtained from placenta derived from the mammals, liver tissues and blood of a hepatopathy patient, fibroblast strains such as MRC-5 cells and IMR-9 cells, strains producing HGF obtained by introducing an expression vector including cDNA encoding hHGF into a host such as CHO cells according to the method described in Japanese Patent Unexamined Publication No. 3-285693 and the like.

Further, as HGF, a precursor protein such as a protein having a signal sequence, a modified protein wherein some of amino acids are replaced, deleted and/or inserted so as not to deteriorate the activity of proliferating hepatocytes, or an altered protein wherein a saccharide is deleted or replaced. Examples of the altered protein include those described in Japanese Patent Unexamined Publication No. 2-288899, WO90/10651, Japanese Patent Unexamined Publication Nos. 3-130091, 3-255096 and 4-30000, Nature, 342, pp.440-443 (1989) and the like.

Examples of HGF preferably used for the lyophilized preparation of the present invention include proteinic factors having the following physicochemical properties. The HGF is preferably derived from human. Examples of particularly preferred HGF include those having the amino acid sequences described in Japanese Patent Unexamined Publication Nos. 3-72883 and 4-89499.
1) The factor has an estimated molecular weight of about 76,000-92,000 by SDS-PAGE (under non-reducing condition);
2) the factor has the activity of proliferating hepatocytes; and
3) the factor has strong affinity for heparin. '
Further, in addition to the above physicochemical properties, preferred HGF has the following properties:
4) The aforementioned activities are inactivated by a heat treatment at 80°C for 10 minutes; and
5) the aforementioned activities are inactivated by digestion with trypsin or chymotrypsin.

Lyophilized HGF preparations containing three ingredients of HGF, a buffering agent and sodium chloride (those described in Japanese Patent Unexamined Publication Nos. 6-247872 and 9-25241: HGF concentration is 5-20 mg/mL) have a problem that, when the content of HGF is reduced to avoid problems such as precipitation of HGF, a favorable cake cannot be obtained in lyophilization process. Further, there is also a problem that aggregation, cloudiness and gelation are observed in an aqueous solution obtained by redissolving a lyophilized preparation obtained from the above three ingredients, and thus sufficient physicochemical stability cannot be attained. Therefore, to prepare a lyophilized preparation that can give a favorable cake form by lyophilization and enables production of an aqueous solution having excellent long-term storage stability, it is essential to add an additive to improve a cake forming property and storage stability in the state of an aqueous solution.

The lyophilized preparation of the present invention is manufactured from an aqueous solution containing HGF at a concentration lower than 5 mg/mL and/or prepared so that an aqueous solution produced from the lyophilized preparation contains HGF at a concentration lower than 5 mg/mL. Preferably, the lyophilized preparation can be manufactured so that an aqueous solution before lyophilization and/or an aqueous solution obtained after redissolution have a pH desirable as an injection and have substantial isotonicity with living bodies or an osmotic pressure ratio acceptable as an injection (1 to 2). The lyophilized preparation of the present invention is characterized to have excellent storage stability. The lyophilized preparation is also characterized in that the preparation can form a favorable lyophilization cake in lyophilization process, and that an aqueous solution obtained by redissolving the lyophilized preparation is free from a problem of aggregation, cloudiness or gelation, thereby sufficient physicochemical stability is achieved. Furthermore, in clinical applications, the preparation can sufficiently exert desired pharmacological actions.

Examples of the stabilizing agent include arginine, lysine, histidine, glutamic acid, aspartic acid, and pharmacologically acceptable salts thereof. Examples of the pharmacologically acceptable salts include alkali metal salts such as sodium salts and potassium salts. These stabilizing agents may be used as a combination of two or more kinds. Examples of preferred stabilizing agents include arginine, lysine, histidine, glutamic acid, aspartic acid and the like. Among them, arginine, lysine, histidine and a combination thereof are particularly preferred. The amount of the stabilizing agent to be added is not particularly limited as long as the storage stability of HGF can be achieved, but is preferably 0.01-100 times by weight, most preferably 0.1-30 times by weight based on the weight of HGF.

The buffering agent is not also particularly limited so long as the agent has an action for adjusting pH of the aqueous solutions before lyophilization and after redissolution and maintaining solubility of HGF. For example, a phosphate buffer, a citrate buffer, an acetate buffer or the like can be used. As the buffering agent, a phosphate buffer, particularly preferably, a sodium phosphate buffer can be preferably used. The amount of the buffering agent to be added is, for example, about 1-100 mM based on the amount of water after redissolution.

Sodium chloride improves the solubility of HGF in the aqueous solutions before lyophilization and after redissolution, however, it is not preferred to add sodium chloride more than necessary, because it increases osmotic pressure. In general, it is sufficient to add sodium chloride in an amount sufficient to achieve an isotonic osmotic pressure with living bodies. The osmotic pressure ratio is most preferably 1-2, which is acceptable as the osmotic pressure ratio of an injection. For example, it is preferable to add 140 mM of sodium chloride based on the volume of water after redissolution.

HGF has a problem that its solubility is rapidly decreased at neutral pH, since the pH overlaps with the isoelectric point of HGF (pI = 7-8). For example, HGF has low solubility of a little less than 1.0 mg/mL around pH 7.0-7.5 in 10 mM sodium phosphate buffer (PBS, room temperature) containing 140 mM sodium chloride. Whilst HGF has a solubility of 5 mg/mL or higher around pH 5.0, and the solubility of HGF becomes higher at a lower pH. Further, at a sodium chloride concentration of 0.14 M, the solubility of HGF is about 1 mg/mL, and when the concentration is made 0.3 M or higher, HGF is dissolved at a concentration of 5 mg/mL or higher. Therefore, it is also conceived that, to increase the solubility of HGF, the solution is kept in an acidic condition at a pH of 5 or lower or the sodium chloride concentration is increased to 0.3 M or higher. In the preparation of the present invention, it is preferred that pH of the aqueous solutions before lyophilization and/or after redissolution is adjusted to be within a weakly acidic range, specifically at a pH of 4.0-6.5, preferably a pH of 5.0-6.5. In such a pH range, formation of a aggregate is suppressed.

The lyophilized HGF preparation of the present invention is preferably added further with a surface active agent. HGF is easily adsorbed to a container material such as glass or a resin. At a low concentration, in particular, adsorption of HGF to a container leads to decrease of a drug content in a solution to be administered. By adding a surface active agent, adsorption of HGF to a container after redissolution can be prevented. Examples of the surface active agent include nonionic surface active agents such as Polysorbate 80, Polysorbate 20, HCO-40, HCO-60, Pluronic F-68 and polyethylene glycol, and a combination of two or more kinds of these agents may also be used. As the surface active agent, polyoxyethylene ether surface active agents (Polysorbate 80 and the like) can be most preferably used. The amount of the surface active agent is, for example, in a range of 0.001-2.0% by weight based on the weight of water after redissolution.

The lyophilized HGF preparation of the present invention can be produced by lyophilizing an aqueous solution containing HGF according to a conventional method. For example, HGF, a stabilizing agent, sodium chloride and a buffering agent can be dissolved in distilled water for injection, optionally added with a surface active agent, sterilized by filtration and introduced into a container such as a vial or an ampoule, and then subjected to lyophilization. The lyophilized HGF preparation of the present invention may contain other additives necessary for formulation, for example, antioxidants, preservatives, excipients, soothing agents and the like. An example of the lyophilization method includes, for example, a method comprising three unit operations: (1) a freezing step for chilling and freezing under atmospheric pressure, (2) a primary drying step for sublimating and drying free water not restrained by a solute under reduced pressure, and (3) a secondary drying step for removing adsorbed water or crystal water intrinsic to the solute (Pharm. Tech. Japan, 8 (1), pp.75-87, 1992). However, the method for producing the lyophilized preparation of the present invention is not limited to the above method. The lyophilized preparation of the present invention can be dissolved by adding a solvent such as distilled water for injection upon use so that the HGF concentration becomes lower than 5 mg/mL.

### Examples

The present invention will be explained more specifically with reference to the following examples. However, the scope of the present invention is not limited to these examples.

### Example 1: Preparation of a lyophilized low-concentration HGF preparation

### (Comparative Example)

HGF is dissolved at a concentration of 1 mg/mL in 10 mM phosphate buffer (pH 6.5) containing 140 mM sodium chloride and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the conditions shown in Table 1. In the table, "→" indicates that temperature is changed.

**Table 1**

| | Freezing process | | Primary drying process | | Secondary drying process | |
|---|---|---|---|---|---|---|
| Temperature (°C) | 20 → -40 | -40 | -40 → -20 | -20 | -20 → 20 | 20 |
| Time (Hr) | 1 | 5 | 3 | 48 | 2 | 24 |
| Pressure (mmHg) | 760 | 760 | < 1 | < 1 | < 1 | < 1 |

### Example 2: Preparation of a lyophilized low-concentration HGF preparation (Comparative Example)

HGF is dissolved with heating at a concentration of 5 mg/mL in 10 mM phosphate buffer (pH 6.5) containing 140 mM sodium chloride and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the conditions shown in Table 1.

### Example 3: Preparation of a lyophilized high-concentration HGF preparation (Comparative Example)

HGF is dissolved at a concentration of 10 mg/mL in 10 mM phosphate buffer (pH 6.5) containing 140 mM sodium chloride, 100 mM arginine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized high-concentration HGF preparation can be obtained by lyophilizing the solution according to the conditions shown in Table 1.

### Example 4: Preparation of a lyophilized low-concentration HGF preparation (Comparative Example)

HGF is dissolved at a concentration of 1 mg/mL in 10 mM citrate buffer (pH 5.0) containing 300 mM sodium chloride and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the conditions shown in Table 1.

### Example 5: Preparation of a lyophilized low-concentration HGF preparation (Comparative Example)

HGF is dissolved at a concentration of 1 mg/mL in 10 mM citrate buffer (pH 5.0) containing 300 mM sodium chloride, 5% glycine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the conditions shown in Table 1.

### Example 6: Preparation of a lyophilized low-concentration HGF preparation (Comparative Example)

HGF is dissolved at a concentration of 1 mg/mL in 10 mM citrate buffer (pH 5.0) containing 300 mM sodium chloride, 5% alanine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the conditions shown in Table 1.

### Example 7: Preparation of a lyophilized low-concentration preparation (Present Invention)

HGF is dissolved at a concentration of 1 mg/mL in 10 mM phosphate buffer (pH 6.5) containing 140 mM sodium chloride and 100 mM arginine and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1. Upon use, this preparation can be dissolved in 2 mL of distilled water for injection to obtain an injection containing HGF at a concentration of 1 mg/mL and having a pH and osmotic pressure ratio (1.5, almost isotonic) acceptable as an injection.

### Example 8: Preparation of a lyophilized low-concentration preparation (Present Invention)

HGF is dissolved at a concentration of 1 mg/mL in 10 mM phosphate buffer (pH 6.5) containing 140 mM sodium chloride, 100 mM arginine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1.

### Example 9: Preparation of a lyophilized low-concentration preparation (Present Invention)

HGF is dissolved at a concentration of 2 mg/mL in 10 mM phosphate buffer (pH 6.5) containing 140 mM sodium chloride, 100 mM arginine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1.

### Example 10: Preparation of a lyophilized low-concentration preparation (Present Invention)

HGF is dissolved at a concentration of 3 mg/mL in 10 mM phosphate buffer (pH 6.5) containing 140 mM sodium chloride, 100 mM arginine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1.

### Example 11: Preparation of a lyophilized low-concentration preparation (Present Invention)

HGF is dissolved at a concentration of 4 mg/mL in 10 mM phosphate buffer (pH 6.5) containing 140 mM sodium chloride, 100 mM arginine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1.

### Example 12: Preparation of a lyophilized low-concentration preparation (Comparative Example)

HGF is dissolved at a concentration of 5 mg/mL in 10 mM phosphate buffer (pH 6.5) containing 140 mM sodium chloride, 100 mM arginine and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1.

### Example 13: Preparation of a lyophilized low-concentration preparation (Present Invention)

A lyophilized low-concentration HGF preparation can be obtained by dissolving HGF at a concentration of 1 mg/mL in the same manner as in Example 8 by using 10 mM phosphate buffer (pH 6.0) instead of 10 mM phosphate buffer (pH 6.5).

### Example 14: Preparation of a lyophilized low-concentration preparation (Present Invention)

A lyophilized low-concentration HGF preparation can be obtained by dissolving HGF at a concentration of 1 mg/mL in the same manner as in Example 8 by using 10 mM phosphate buffer (pH 5.5) instead of 10 mM phosphate buffer (pH 6.5).

### Example 15: Preparation of a lyophilized low-concentration preparation (Present Invention)

A lyophilized low-concentration HGF preparation can be obtained by dissolving HGF at a concentration of 1 mg/mL in the same manner as in Example 8 by using 10 mM phosphate buffer (pH 5.0) instead of 10 mM phosphate buffer (pH 6.5).

### Example 16: Preparation of a lyophilized low-concentration preparation (Present Invention)

A lyophilized low-concentration HGF preparation can be obtained by dissolving HGF at a concentration of 1 mg/mL in the same manner as in Example 8 by using 10 mM phosphate buffer (pH 7.2) instead of 10 mM phosphate buffer (pH 6.5).

### Example 17: Preparation of a lyophilized low-concentration preparation (Present Invention)

A lyophilized low-concentration HGF preparation can be obtained by dissolving HGF at a concentration of 1 mg/mL in the same manner as in Example 8 by using 10 mM phosphate buffer (pH 7.0) instead of 10 mM phosphate buffer (pH 6.5).

### Example 18: Preparation of a lyophilized low-concentration preparation (Present Invention)

A lyophilized low-concentration HGF preparation can be obtained in the same manner as in Example 8 by using 50 mM arginine instead of 100 mM arginine.

### Example 19: Preparation of a lyophilized low-concentration preparation (Present Invention)

A lyophilized low-concentration HGF preparation can be obtained in the same manner as in Example 8 by using lysine instead of arginine.

### Example 20: Preparation of a lyophilized low-concentration preparation (Present Invention)

A lyophilized low-concentration HGF preparation can be obtained in the same manner as in Example 8 by using histidine instead of arginine.

### Example 21: Preparation of a lyophilized low-concentration preparation

A lyophilized low-concentration HGF preparation can be obtained in the same manner as in Example 8 by using glutamine instead of arginine.

### Example 22: Preparation of a lyophilized low-concentration preparation

A lyophilized low-concentration HGF preparation can be obtained in the same manner as in Example 8 by using cysteine instead of arginine.

### Example 23: Preparation of a lyophilized low-concentration preparation

A lyophilized low-concentration HGF preparation can be obtained in the same manner as in Example 8 by using proline instead of arginine.

### Example 24: Preparation of a lyophilized low-concentration preparation (Present Invention)

A lyophilized low-concentration HGF preparation can be obtained in the same manner as in Example 8 by using sodium glutamate instead of arginine.

### Example 25: Preparation of a lyophilized low-concentration preparation (Present Invention)

A lyophilized low-concentration HGF preparation can be obtained in the same manner as in Example 8 by using sodium aspartate instead of arginine.

### Example 26: Preparation of a lyophilized low-concentration preparation

A lyophilized low-concentration HGF preparation can be obtained in the same manner as in Example 8 by using glycine instead of arginine.

### Example 27: Preparation of a lyophilized low-concentration preparation (Present Invention)

A lyophilized low-concentration HGF preparation can be obtained in the same manner as in Example 8 by using a charging amount of 5 mL each instead of 2 mL.

### Example 28: Preparation of a lyophilized low concentration preparation

Sodium dextran sulfate and HGF are dissolved at concentrations of 50 mg/mL and 1 mg/mL, respectively, in 10 mM sodium phosphate buffer (pH 6.5) containing 140 mM sodium chloride and 0.01% Polysorbate 80 and subjected to filtration for sterilization to obtain an aqueous HGF solution. After pH of the aqueous solution is adjusted, the solution is aseptically charged into vials in an amount of 2 mL per vial. A lyophilized low-concentration HGF preparation can be obtained by lyophilizing the solution according to the same conditions as in Example 1.

### Test Example 1: Evaluation of solubility of HGF

### (1) Method for evaluating solubility of HGF

HGF was weighed in a polypropylene tube and added with 10 mM sodium phosphate buffer containing sodium chloride and a stabilizing agent at various concentrations and 0.01% Polysorbate 80. The tube was immediately maintained at a constant temperature to dissolve HGF. Immediately after the dissolution, the solution was subjected to centrifugation (15,000 rpm, 10 minutes, constant temperature) to completely separate the saturated HGF solution and undissolved HGF. The supernatant was sampled and filtered through a low protein adsorptive filter, Millipore GV (hydrophilic Durapore, 0.22 µm), and HGF concentration of the resulting saturated solution was quantified by HPLC (gel filtration method) to determine solubility of HGF at saturation.
Conditions for HPLC analysis
Column: TOSOH TSK G-3000SWXL (φ 0.78 x 30 cm)
Flow rate: 0.3 mL/min
Detection wavelength: OD 280 nm
Temperature: 30°C
Carrier: 0.3 M NaCl, 50 mM sodium phosphate, 0.1% SDS, pH 7.5
Application: 50 µl
Retention time of HGF: 24.0 min

### (2) Influence of pH on solubility of HGF

Solutions of different pH were prepared by using 10 mM sodium phosphate buffer containing 140 mM sodium chloride and 0.01% Polysorbate 80. The solubility of HGF was examined at 4°C and 20°C by the method of (1). The results are shown in Table 2. The solubility of HGF gradually increased with the decrease of pH. Marked improvement of the solubility was found at pH 5.0 or lower. Further, increase of solubility with elevation of temperature was observed in every sample.

**Table 2**

| | 20°C | 4°C |
|---|---|---|
| pH 7.5 | 0.8 | 0.4 |
| pH 7.0 | 1.8 | 1.0 |
| pH 6.0 | 2.3 | 1.3 |
| pH 5.0 | 5.9 | 4.2 |

| | | |
|---|---|---|
| (Solubility of HGF is shown in mg/mL) | | |

### (3) Influences of sodium chloride concentration on solubility of HGF

10 mM Sodium phosphate buffer solutions (pH 7.5) containing sodium chloride at various concentrations and 0.01% Polysorbate 80 were prepared. The solubility of HGF was examined at 4°C and 20°C by the method of (1). The results are shown in Table 3. Remarkable increase of solubility of HGF was observed with the increase of sodium chloride concentration. Further, increase of solubility with elevation of temperature was observed in every sample.

**Table 3**

| | | |
|---|---|---|
| | 20°C | 4°C |
| Not added | 0.3 | 0.1 |
| + 140 mM NaCl | 0.8 | 0.4 |
| + 230 mM NaCl | 3.2 | 1.4 |
| + 300 mM NaCl | 8.5 | 4.0 |
| + 900 mM NaCl | > 190 | - |

| | | |
|---|---|---|
| (Solubility of HGF is indicated in mg/mL) | | |

### (4) Influence of various stabilizing agents on solubility of HGF

Influence of various additives for pharmaceutical preparations on solubility of HGF was examined. HGF was dissolved at a concentration of 1 mg/mL in 10 mM sodium phosphate buffer solutions (pH 6.8-7.5) containing additives at various concentrations, 140 mM sodium chloride and 0.01% Polysorbate 80 to obtain aqueous HGF solutions. An amount of 200 µL of each aqueous solution was introduced into each well of a 96-well microtiter plate and stored at 4°C for 48 hours. Then, turbidity of each aqueous HGF solution was determined by measuring OD at 450 nm using a plate reader. The turbidity of the solution increased with the decrease of the solubility of HGF which resulted in aggregation and precipitation of HGF.

Influence on HGF solubility was evaluated for additives including 20 kinds of L-amino acids (arginine, lysine, histidine, serine, threonine, asparagine, glutamine, sodium aspartate, sodium glutamate, cysteine, glycine, proline, alanine, isoleucine, leucine, methionine, phenylalanine, tyrosine, tryptophan, valine), 7 kinds of saccharides (mannitol, fructose, trehalose, glucose, sorbitol, sucrose, lactose), 3 kinds of polymers (dextran sulfate, dextran, PEG), 3 kinds of proteins (human serum albumin, acidic gelatin, basic gelatin) and 3 kinds of surface active agents (Polysorbate 80, Polysorbate 20, HCO-40, HCO-60). A stabilization effect to maintain the solubility of HGF was observed in the substances listed below.
(i) Amino acids: arginine, lysine, histidine, sodium glutamate, sodium aspartate, glutamine, cysteine, proline (the effect was confirmed at 0.05 M)
(ii) Polysaccharides: dextran sulfate (the effect was confirmed at 0.1%)

By using the amino acids that gave remarkable effects, solutions were prepared in 10 mM sodium phosphate buffer solutions (pH 7.0) which contained 140 mM sodium chloride, 0.01% Polysorbate 80, and each of the amino acids at a variety of concentrations. The solubility of HGF was examined at 4°C by the method of (1). The results are shown in Table 4.

**Table 4**

| | | Saturation solubility | Osmotic pressure ratio |
|---|---|---|---|
| No additive | | 1.0 | 1.0 |
| + L-Arg | 50 mM | 7.3 | 1.3 |
| + L-Lys | 50 mM | 4.5 | 1.3 |
| + L-His | 50 mM | 3.2 | 1.2 |
| + L-GluNa | 50 mM | 2.2 | 1.3 |
| | | | |
| + L-Arg | 100 mM | > 10 | 1.6 |
| + L-Lys | 100 mM | > 10 | 1.6 |
| + L-His | 100 mM | 4.8 | 1.4 |
| + L-GluNa | 100 mM | 3.2 | 1.6 |

| | | | |
|---|---|---|---|
| (Solubility of HGF is shown in mg/mL) | | | |

### Test Example 2: Properties of aqueous HGF solutions before and after lyophilization

To observe any change in physical stability of HGF during the lyophilization process, an aqueous HGF solution before lyophilization and an aqueous HGF solution obtained by redissolving the lyophilized preparation in purified water without any further treatment were stored at 4°C for 24 hours, and a property (cloudiness) of the solutions after dissolution was visually observed. The time required for redissolution of the lyophilized preparation and the osmotic pressure ratio were also evaluated. The results are shown in Table 5.

When the lyophilized preparations of Examples 1 and 22 were redissolved and stored at 4°C for 24 hours, the solutions became cloudy. The preparations of other examples were found to be stable as to the above property.

**Table 5**

| Preparation | Aqueous solution before lyophilization | Aqueous solution after redissolution | Osmotic pressure ratio |
|---|---|---|---|
| Example 1 | Cloudy | Instantly soluble, cloudy | 1.0 |
| Example 4 | Clear | Instantly soluble, clear | 2.0 |
| Example 5 | Clear | Hardly soluble, clear | 4.0 |
| Example 6 | Clear | Hardly soluble, clear | 3.9 |
| Example 8 | Clear | Instantly soluble, clear | 1.5 |
| Example 19 | Clear | Instantly soluble, clear | 1.6 |
| Example 20 | Clear | Instantly soluble, clear | 1.4 |
| Example 21 | Clear | Instantly soluble, clear | 1.3 |
| Example 22 | Clear | Hardly soluble, cloudy | 1.7 |
| Example 23 | Clear | Instantly soluble, clear | 1.3 |
| Example 24 | Clear | Instantly soluble, clear | 1.5 |
| Example 25 | Clear | Instantly soluble, clear | 1.5 |
| Example 26 | Clear | Instantly soluble, clear | 1.3 |
| Example 28 | Clear | Instantly soluble, clear | 1.3 |

### Test Example 3: Properties of lyophilized preparation after dissolution

Time required for redissolution and a solution property (cloudiness) after redissolution of the lyophilized preparations obtained in the examples were evaluated immediately after lyophilization and after storage at 25°C, 40°C and 50°C for 1 month. The lyophilized preparations were dissolved in purified water and the property was evaluated at room temperature. The results are shown in Table 6.

Among those stored at 25°C, the solution of the preparation of Example 22 became cloudy immediately after redissolution of the lyophilized preparation, whilst the preparations of the other examples were found to be stable as to the property. Further, under storage at 40°C and 50°C, the solutions of the preparations of Examples 1, 22, 23, 24 and 25 became cloudy immediately after dissolution. However, the preparations of the other examples were found to be stable as to the above property.

**Table 6**

| Preparation | Preparation after storage for 1 month | | |
|---|---|---|---|
| | 25°C | 40°C | 50°C |
| Example 1 | Instantly soluble, clear | Instantly soluble, cloudy | Instantly soluble, cloudy |
| Example 4 | Instantly soluble, clear | Instantly soluble, clear | Instantly soluble, clear |
| Example 5 | Hardly soluble, clear | Hardly soluble, clear | Hardly soluble, clear |
| Example 6 | Hardly soluble, clear | Hardly soluble, clear | Hardly soluble, clear |
| Example 8 | Instantly soluble, clear | Instantly soluble, clear | Instantly soluble; clear |
| Example 19 | Instantly soluble, clear | Instantly soluble, clear | Instantly soluble, clear |
| Example 20 | Instantly soluble, clear | Instantly soluble, clear | Instantly soluble, clear |
| Example 21 | Instantly soluble, clear | Instantly soluble, clear | Instantly soluble, clear |
| Example 22 | Hardly soluble, cloudy | Hardly soluble, cloudy | Hardly soluble, cloudy |
| Example 23 | Instantly soluble, clear | Instantly soluble, cloudy | Instantly soluble, cloudy |
| Example 24 | Instantly soluble, clear | Instantly soluble, cloudy | Instantly soluble, cloudy |
| Example 25 | Instantly soluble, clear | Instantly soluble, cloudy | Instantly soluble, cloudy |
| Example 26 | Instantly soluble, clear | Instantly soluble, clear | Instantly soluble, clear |
| Example 28 | Instantly soluble, clear | Instantly soluble, clear | Instantly soluble, clear |

### Test Example 4: Change in a aggregate content in a lyophilized preparation

The ratio of a aggregate content and an HGF content in the lyophilized preparations obtained in the examples were compared immediately after lyophilization (initial value) and after storage at 25°C, 40°C and 50°C for 1 month. The method of Test Example 1 (1) (gel filtration method) was applied. The results are shown in Tables 7 and 8.
Retention time of aggregates: 20.4 min, 21.8 min
Retention time of HGF: 24.0 min

With the elevation of the storage temperature, an increasing tendency of aggregate formation was observed. However, the lyophilized preparations of Examples 8, 19 and 20, in particular, gave extremely low aggregate formation and they were found to be physicochemically stable. It was thus concluded that addition of arginine, lysine and histidine kept aggregate formation at a low level even after high-temperature storage (aggregate formation rate: about 3% or lower at 40°C and about 5-9% or lower at 50°C), thereby improved storage stability. A similar test was performed as a comparative example by using a lyophilized preparation of Example 1 which was obtained according to the same method using the same ingredients except that arginine was not contained. As a result, marked increase in aggregate formation was observed with the elevation of the storage temperature.

Further, as shown in Table 8, it was demonstrated that decrease of the HGF concentration (lower than 5 mg/mL) accelerated aggregate formation, which resulted in lower storage stability. It was found that, when glycine or alanine was used as a stabilizing agent of the lyophilized preparation as described in Japanese Patent Unexamined Publication No. 9-25411, aggregate formation was accelerated and storage stability was lowered in the lyophilized low-concentration HGF preparations (Examples 5, 6 and 26, 1 mg/mL) as compared to the lyophilized high-concentration HGF preparations (Examples 5 and 6 in Japanese Patent Unexamined Publication No. 9-25411, 20 mg/mL).

Whilst it was demonstrated that, in the lyophilized preparations of Examples 8, 19 and 20 wherein arginine, lysine or histidine was used as a stabilizing agent of the lyophilized preparation, aggregate formation was markedly suppressed and thus storage stability was improved even in the lyophilized low-concentration HGF preparations (1 mg/mL).

**Table 7**

| Preparation | Preparation after storage for 1 month | | | |
|---|---|---|---|---|
| | Initial value | 25°C | 40°C | 50°C |
| Example 1 | 0.48 | 5.50 | 24.27 | 40.63 |
| Example 4 | 0.35 | 0.48 | 3.80 | 11.24 |
| Example 5 | 0.31 | 0.69 | 4.40 | 9.58 |
| Example 6 | 0.30 | 0.54 | 3.20 | 9.53 |
| Example 8 | 0.30 | 0.11 | 0.18 | 0.60 |
| Example 19 | 0.31 | 0.16 | 1.74 | 4.48 |
| Example 20 | 0.31 | 0.18 | 0.28 | 0.88 |
| Example 21 | 0.31 | 0.54 | 2.77 | 16.89 |
| Example 22 | - | - | - | - |
| Example 23 | 0.32 | 0.31 | 3.24 | 11.24 |
| Example 24 | 0.32 | 2.19 | 4.90 | 6.39 |
| Example 25 | 0.34 | 1.11 | 4.80 | 7.73 |
| Example 26 | 0.36 | 0.33 | 6.21 | 22.66 |
| Example 28 | 2.74 | 3.48 | 13.30 | 32.87 |
| Example 1* | 1.07 | | | 6.17 |
| Example 5* | 0.92 | | | 4.09 |
| Example 6* | 0.93 | | | 2.90 |
| Example 9* | 1.78 | | | 14.01 |

| | | | | |
|---|---|---|---|---|
| Quoted from Tables 4 and 6 in Japanese Patent Unexamined Publication No. 9-25241 | | | | |

**Table 8**

| | Preparation | Aggregate content/HGF content of lyophilized preparation stored at 50°C for 1 month |
|---|---|---|
| | [Preparation containing no amino acid] | |
| Example 1 | (HGF at 1 mg/mL) | 40.63 |
| Example 4 | (HGF at 1 mg/mL) | 11.24 |
| Example 9' | (HGF at 10 mg/mL) | 14.01 |
| Example 1' | (HGF at 20 mg/mL) | 6.17 |

| | [Preparation containing glycine] | |
|---|---|---|
| Example 5 | (HGF at 1 mg/mL) | 9.58 |
| Example 5* | (HGF at 20 mg/mL) | 4.09 |

| | [Preparation containing alanine] | |
|---|---|---|
| Example 6 | (HGF at 1 mg/mL) | 9.53 |
| Example 6* | (HGF at 20 mg/mL) | 2.90 |

| | | |
|---|---|---|
| * Quoted from Tables 4 and 6 in Japanese Patent Unexamined Publication No. 9-25241 | | |

### Test Example 5: Change in aggregate content in lyophilized preparation - influence of pH on aggregate formation

The ratio of the aggregate content and the HGF content in the lyophilized preparations having various pH prepared in Examples 8, 13, 14, 16 and 17 was determined immediately after lyophilization (initial value) and after storage at 50°C for 1 month, 2 months and 3 months. The method of Test Example 1 (1) (gel filtration method) was used. The results are shown in Table 9.
Retention time of aggregate: 20.4 min, 21.8 min
Retention time of HGF: 24.0 min

In the lyophilized preparations of Examples 16 and 17 having pH of 7.0 and 7.2, aggregate formation was increased with time during the storage at 50°C. Whilst in the preparations of Example 8, 13 and 14, which had pH of 6.5 or lower, aggregate formation was kept at a low level. It was thus concluded that the stability was improved under weakly acidic pH.

**Table 9**

| Preparation | Stored at 50°C | | | |
|---|---|---|---|---|
| | Initial value | Stored for 1 month | Stored for 2 months | Stored for 3 months |
| Example 14 (pH 5.5) | 0.48 | 0.38 | 0.56 | 0.76 |
| Example 13 (pH 6.0) | 0.35 | 0.91 | 0.51 | 1.31 |
| Example 8 (pH 6.5) | 0.31 | 1.40 | 1.58 | 1.28 |
| Example 17 (pH 7.0) | 0.30 | 1.26 | 2.16 | 2.96 |
| Example 16 (pH 7.2) | 0.30 | 5.64 | 7.63 | 12.71 |

### Test Example 6: Change in biological activity (specific activity) of lyophilized preparation

The lyophilized preparations prepared in Examples 1 and 8 were stored at 25°C or 50°C for 2 months or at 10°C or 25°C for 1.5 years. The biological activities of aqueous solutions obtained by redissolving the lyophilized preparations were determined by the method for determining biological activity shown below. The results are shown in Table 10.

### Method for determining biological activity

A human hepatic cell strain PLC/PRF/5 was cultured until the logarithmic growth phase, and the cell survival rate was observed. Then, a cell solution was prepared at a density of 0.7 × 10⁵ cells/mL. An amount of 100 µL of the cell solution was put into each well of a 96-well assay plate added beforehand with an HGF sample or a standard sample so that the cell count was 0.7 × 10⁴ cells/well (n = 4). After pre-incubation at 37°C for 20 hours in a 5% carbon dioxide incubator, [³H-thymidine] was added and the culture was further continued for 6 hours. After completion of the culture, cells were collected by using a Beta Plate System (Pharmacia) and the amount of [³H] taken up into the cells was measured. The measurement results were verified by the parallel line test. The titer (%) was obtained by dividing the specific activity of the HGF sample by the specific activity of the standard.

As for the lyophilized preparation of Example 8 added with arginine, the biological activity was almost unchanged even after the high-temperature storage and the preparation was stable as for the biological activity. Further, a similar test was performed as a comparative example by using a lyophilized preparation obtained by the same method with the same ingredients as in Example 1 except that arginine was not contained. As a result, a marked decrease in biological activity was observed with the elevation of the storage temperature.

**Table 10**

| | Stored for 2 months | | Stored for 1.5 years | |
|---|---|---|---|---|
| | 25°C | 50°C | 10°C | 25°C |
| Example 1 | 64.5% | 10.2% | - | - |
| Example 8 | 87.1% | 71.0% | 72.6% | 66.1% |

### Industrial Applicability

The lyophilized preparation of the present invention can be used to prepare a clinically useful aqueous solution containing HGF at a low concentration, and said preparation is almost free from aggregate formation during lyophilization and storage after the lyophilization and thus has excellent stability. Further, said preparation is characterized by favorable cake forming property during lyophilization and excellent re-solubility. In addition, said preparation can also be made into a preparation having a pH and an osmotic pressure ratio desirable as an injection.

## Claims

1. A lyophilized preparation comprising a hepatocyte growth factor, a stabilising agent selected from the group consisting of arginine, lysine, histidine, glutamic acid, aspartic acid and a pharmacologically acceptable salt thereof for preventing formation of an aggregate of the hepatocyte growth factor, sodium chloride, and a buffering agent, **characterized in that** the lyophilized preparation is prepared from an aqueous solution containing the hepatocyte growth factor at a concentration lower than 5 mg/mL and/or the lyophilized preparation is used for preparing an aqueous solution containing the hepatocyte growth factor at a concentration lower than 5 mg/mL by redissolution.

2. The lyophilized preparation according to claim 1, wherein the stabilizing agent is selected from the group consisting of arginine, lysine, histidine, and a pharmacologically acceptable salt thereof.

3. The lyophilized preparation according to claim 1 or 2, wherein the buffering agent is a phosphoric acid salt.

4. The lyophilized preparation according to any one of claims 1 to 3, wherein a pH of the aqueous solution before lyophilization is in the range of 5 to 6.5

5. The lyophilized preparation according to any one of claims 1 to 3, wherein a pH of the aqueous solution before redissolution is in the range of 5 to 6.5

6. The lyophilized preparation according to any one of claims 1 to 5, which further contains a surface active agent.

7. The lyophilized preparation according to claim 6, wherein the surface active agent is a nonionic surface active agent.

8. The lyophilized preparation according to claim 7, wherein the nonionic surface active agent is a polyoxyethylene ether surface active agent.

9. The lyophilized preparation according to any one of claims 1 to 8, which is prepared in a vial or an ampoule.

10. The lyophilized preparation according to any one of claims 1 to 9, which contains the stabilizing agent in an amount of 0.1 to 30 times by weight based on the weight of HGF.

11. Use of a stabilizing agent for HGF to lyophilize an aqueous solution containing HGF at a concentration lower than 5 mg/mL, having a pH in the range of 5 to 6.5, which is selected from the group consiting of arginine, lysine, histidine, glutamic acid, aspartic acid, and a pharmacologically acceptable salt thereof.

12. The use of a stabilizing agent according to claim 11, for preventing HGF aggregate formation during lyophilization and/or storage after the lyophilization.

13. The use of a stabilizing agent according to claim 11, in an amount of 0.1 to 30 times by weight based on the weight of HGF.

14. The use of a stabilizing agent according to any one of claims 11 to 13, wherein the agent is selected from the group consisting of arginine, lysine, histidine, glutamic acid, aspartic acid, and a pharmacologically acceptable salt thereof.

15. A method for preparing a lyophilized preparation comprising a hepatocyte growth factor by subjecting an aqueous solution having a pH in the range of 5.0 to 6.5 and comprising a hepatocyte growth factor, a stabilizing agent for preventing formation of an aggregate of the hepatocyte growth factor, sodium chloride, and a buffering agent to lyophilization, **characterized in that** the aqueous solution comprises the hepatocyte growth factor at a concentration lower than 5 mg/mL.

16. The method according to claim 15, wherein the stabilizing agent is selected from the group consisting of arginine, lysine, histidine, glutamic acid, aspartic acid, and a pharmacologically acceptable salt thereof.

17. The method according to claim 15 or 16, wherein the stabilizing agent is selected from the group consisting of arginine, lysine, histidine, and a pharmacologically acceptable salt thereof.

18. The method according to any one of claims 15 to 17, wherein the buffering agent is a phosphoric acid salt.

19. The method according to any one of claims 15 to 18, wherein the aqueous solution further contains a surface active agent.

20. The method according to claim 19, wherein the surface active agent is a nonionic surface active agent.

21. The method according to claim 20, wherein the nonionic surface active agent is a polyoxyethylene ether surface active agent.

22. The method according to any one of claims 15 to 21, which is carried out in a vial or an ampoule.

23. The method according to claims 15 to 22, wherein the preparation comprises the stabilizing agent in an amount of 0.1 to 30 times by weight based on the weight of HGF.

## Patentansprüche

1. Ein lyophilisiertes Präparat enthaltend einen Hepatocyten-Wachstumsfaktor, ein Stabilisierungsmittel ausgewählt aus der Gruppe bestehend aus Arginin, Lysin, Histidin, Glutaminsäure, Asparaginsäure und ein pharmazeutisch akzeptables Salz davon zur Vermeidung der Bildung von Aggregaten des Hepatocyten-Wachstumsfaktors, Natriumchlorid, und eine Puffersubstanz, **dadurch gekennzeichnet, dass** das lyophilisierte Präparat aus einer wässrigen Lösung hergestellt wird, die den Hepatocyten-Wachstumsfaktor in einer Konzentration von weniger als 5 mg/mL enthält und/oder dass das lyophilisierte Präparat verwendet wird zur Herstellung einer wässrigen Lösung enthaltend den Hepatocyten-Wachstumsfaktor in einer Konzentration von weniger als 5 mg/mL durch Wiederauflösung.

2. Das lyophilisierte Präparat gemäß Anspruch 1, wobei das Stabilisierungsmittel ausgewählt ist aus der Gruppe bestehend aus Arginin, Lysin, Histidin, und ein pharmakologisch akzeptables Salz davon.

3. Das lyophilisierte Präparat gemäß Anspruch 1 oder 2, wobei die Puffersubstanz ein Phosphorsäuresalz ist.

4. Das lyophilisierte Präparat gemäß irgend einem der Ansprüche 1 bis 3, wobei der pH-Wert der wässrigen Lösung vor der Lyophilisierung im Bereich von 5 bis 6,5 eingestellt wird.

5. Das lyophilisierte Präparat gemäß irgend einem der Ansprüche 1 bis 3, wobei der pH-Wert der wässrigen Lösung vor der Wiederauflösung im Bereich von 5 bis 6,5 eingestellt wird.

6. Das lyophilisierte Präparat gemäß irgend einem der Ansprüche 1 bis 5, das weiterhin eine oberflächenaktive Substanz enthält.

7. Das lyophilisierte Präparat gemäß Anspruch 6, wobei die oberflächenaktive Substanz eine nichtionische oberflächenaktive Substanz ist.

8. Das lyophilisierte Präparat gemäß Anspruch 7, wobei die nichtionische oberflächenaktive Substanz eine Polyoxyethylen-Ether oberflächenaktive Substanz ist.

9. Das lyophilisierte Präparat gemäß irgend einem der Ansprüche 1 bis 8, das in einem Gefäß oder in einer Ampulle hergestellt wird.

10. Das lyophilisierte Präparat gemäß irgend einem der Ansprüche 1 bis 9, wobei das Stabilisierungsmittel in einem gewichtsmäßigen Verhältnis vom 0,1 bis 30-fachen bezogen auf das Gewicht des HGF enthalten ist.

11. Verwendung eines Stabilisierungsmittels für HGF zur Lyophilisierung einer wässrigen Lösung enthaltend HGF in einer Konzentration von weniger als 5 mg/mL, mit einem pH-Wert im Bereich von 5 bis 6,5, das ausgewählt ist aus der Gruppe bestehend aus Arginin, Lysin, Histidin, Glutaminsäure, Asparaginsäure, und ein pharmakologisch akzeptables Salz davon.

12. Die Verwendung eines Stabiliserungsmittels gemäß Anspruch 11 zur Vermeidung der Bildung von HGF-Aggregaten während der Lyophilisierung und/oder Lagerung nach der Lyophilisierung.

13. Die Verwendung des Stabilisierungsmittels gemäß Anspruch 11, in einem gewichtsmäßigen Verhältnis vom 0,1 bis 30-fachen bezogen auf das Gewicht des HGF.

14. Die Verwendung des Stabilisierungsmittels gemäß irgend einem der Ansprüche 11 bis 13, wobei das Mittel ausgewählt ist aus der Gruppe bestehend aus Arginin, Lysin, Histidin, Glutaminsäure, Asparaginsäure, und ein pharmakologisch akzeptables Salz davon.

15. Ein Verfahren zur Herstellung eines lyophilisierten Präparates enthaltend einen Hepatocyten-Wachstumsfaktor durch Lyophilisieren einer wässrigen Lösung mit einem pH-Wert im Bereich von 5,0 bis 6,5 und enthaltend einen Hepatocyten-Wachtumsfaktor, ein Stabilisierungsmittel zur Vermeidung von Aggregaten des Hepatocyten-Wachstumsfaktors, Natriumchlorid, und eine Puffersubstanz, **dadurch gekennzeichnet, dass** die wässrige Lösung den Hepatocyten-Wachstumsfaktor in einer Konzentration von weniger als 5 mg/mL enthält.

16. Das Verfahren gemäß Anspruch 15, wobei das Stabilisierungsmittel ausgewählt ist aus der Gruppe bestehend aus Arginin, Lysin, Histidin, Glutaminsäure, Asparaginsäure, und ein pharmakologisch akzeptables Salz davon.

17. Das Verfahren gemäß Anspruch 15 oder 16, wobei das Stabilisierungsmittel ausgewählt ist aus der Gruppe bestehend aus Arginin, Lysin, Histidin, und ein pharmakologisch akzeptables Salz davon.

18. Das Verfahren gemäß irgend einem der Ansprüche 15 bis 17, wobei die Puffersubstanz ein Phosphorsäuresalz ist.

19. Das Verfahren gemäß irgend einem der Ansprüche 15 bis 18, wobei die wässrige Lösung weiterhin eine oberflächenaktive Substanz enthält.

20. Das Verfahren gemäß Anspruch 19, wobei die oberflächenaktive Substanz eine nichtionische oberflächenaktive Substanz ist.

21. Das Verfahren gemäß Anspruch 20, wobei die nichtionische oberflächenaktive Substanz eine Polyoxyethylen-Ether oberflächenaktive Substanz ist.

22. Das Verfahren gemäß irgend einem der Ansprüche 15 bis 21, wobei das Verfahren in einem Gefäß oder einer Ampulle durchgeführt wird.

23. Das Verfahren gemäß den Ansprüchen 15 bis 22, wobei das Präparat das Stabilisierungsmittel in einem gewichtsmäßigen Verhältnis vom 0,1 bis 30-fachen bezogen auf das Gewicht des HGF enthält.

## Revendications

1. Préparation lyophilisée comprenant un facteur de croissance des hépatocytes, un agent stabilisant choisi dans le groupe constitué par l'arginine, la lysine, l'histidine, l'acide glutamique, l'acide aspartique et un sel pharmacologiquement acceptable de ceux-ci pour la prévention de la formation d'un agrégat du facteur de croissance des hépatocytes ; du chlorure de sodium, et un agent tampon, **caractérisée en ce que** la préparation lyophilisée est préparée à partir d'une solution aqueuse contenant le facteur de croissance des hépatocytes à une concentration inférieure à 5 mg/mL et/ou la préparation lyophilisée est utilisée pour la préparation d'une solution aqueuse contenant le facteur de croissance des hépatocytes à une concentration inférieure à 5 mg/mL par redissolution.

2. Préparation lyophilisée selon la revendication 1, dans laquelle l'agent stabilisant est choisi dans le groupe constitué par l'arginine, la lysine, l'histidine, et un sel pharmacologiquement acceptable de celles-ci.

3. Préparation lyophilisée selon la revendication 1 ou 2, dans laquelle l'agent tampon est un sel de l'acide phosphorique.

4. Préparation lyophilisée selon l'une quelconque des revendications 1 à 3, dans laquelle un pH de la solution aqueuse avant la lyophilisation se trouve dans la plage de 5 à 6,5.

5. Préparation lyophilisée selon l'une quelconque des revendications 1 à 3, dans laquelle un pH de la solution aqueuse avant la redissolution se trouve dans la plage de 5 à 6,5.

6. Préparation lyophilisée selon l'une quelconque des revendications 1 à 5, qui contient en outre un agent tensioactif.

7. Préparation lyophilisée selon la revendication 6, dans laquelle l'agent tensioactif est un agent tensioactif non ionique.

8. Préparation lyophilisée selon la revendication 7, dans laquelle l'agent tensioactif non ionique est un agent tensioactif à base d'éther de polyoxyéthylène.

9. Préparation lyophilisée selon l'une quelconque des revendications 1 à 8, qui est préparée dans un flacon ou une ampoule.

10. Préparation lyophilisée selon l'une quelconque des revendications 1 à 9, qui contient l'agent stabilisant en une quantité de 0,1 à 30 fois en poids par rapport au poids du HGF.

11. Utilisation d'un agent stabilisant pour le HGF pour lyophiliser une solution aqueuse contenant le HGF à une concentration inférieure à 5 mg/mL, ayant un pH dans la plage de 5 à 6,5, qui est choisi dans le groupe constitué par l'arginine, la lysine, l'histidine, l'acide glutamique, l'acide aspartique, et un sel pharmacologiquement acceptable de ceux-ci.

12. Utilisation d'un agent stabilisant selon la revendication 11, pour la prévention de la formation d'agrégats du HGF durant la lyophilisation et/ou le stockage après la lyophilisation.

13. Utilisation d'un agent stabilisant selon la revendication 11, en une quantité de 0,1 à 30 fois en poids par rapport au poids du HGF.

14. Utilisation d'un agent stabilisant selon l'une quelconque des revendications 11 à 13, dans laquelle l'agent est choisi dans le groupe constitué par l'arginine, la lysine, l'histidine, l'acide glutamique, l'acide aspartique, et un sel pharmacologiquement acceptable de ceux-ci.

15. Procédé de préparation d'une préparation lyophilisée comprenant un facteur de croissance des hépatocytes par la soumission d'une solution aqueuse ayant un pH dans la plage de 5,0 à 6,5 et comprenant un facteur de croissance des hépatocytes, un agent stabilisant pour la prévention de la formation d'un agrégat du facteur de croissance des hépatocytes, du chlorure de sodium, et un agent tampon à une lyophilisation, **caractérisé en ce que** la solution aqueuse comprend le facteur de croissance des hépatocytes à une concentration inférieure à 5 mg/mL.

16. Procédé selon la revendication 15, dans lequel l'agent stabilisant est choisi dans le groupe constitué par l'arginine, la lysine, l'histidine, l'acide glutamique, l'acide aspartique, et un sel pharmacologiquement acceptable de ceux-ci.

17. Procédé selon la revendication 15 ou 16, dans lequel l'agent stabilisant est choisi dans le groupe constitué par l'arginine, la lysine, l'histidine, et un sel pharmacologiquement acceptable de ceux-ci.

18. Procédé selon l'une quelconque des revendications 15 à 17, dans lequel l'agent tampon est un sel de l'acide phosphorique.

19. Procédé selon l'une quelconque des revendications 15 à 18, dans lequel la solution aqueuse contient en outre un agent tensioactif.

20. Procédé selon la revendication 19, dans lequel l'agent tensioactif est un agent tensioactif non ionique.

21. Procédé selon la revendication 20, dans lequel l'agent tensioactif non ionique est un agent tensioactif à base d'éther de polyoxyéthylène.

22. Procédé selon l'une quelconque des revendications 15 à 21, qui est réalisé dans un flacon ou une ampoule.

23. Procédé selon les revendications 15 à 22, dans lequel la préparation comprend l'agent stabilisant en une quantité de 0,1 à 30 fois en poids par rapport au poids du HGF.
